# EUROPEAN PATENT APPLICATION

(11) **EP 4 782 454 A1**
(43) Date of publication of application: **29.07.2026**
(21) Application number: 26154126.2
(22) Date of filing: 26.01.2026
(51) Int. Cl.: C07D 513/04, A61P 35/00, A61K 31/4365

(54) **4,6-DIMETHYLISOTHIAZOLO[5,4-B]PYRIDINE DERIVATIVES COUPLED WITH AN N-ACETYLHYDRAZONE MOIETY**

(30) Priority: 24.01.2025 PL 45103525
(71) Applicant: Uniwersytet Medyczny Im. Piastów Slaskich We Wroclawiu, 50-367 Wroclaw (PL)
(72) Inventor: Strzelecka, Malgorzata, 53-111 Wroclaw (PL); Swiatek, Piotr, 55-120 Pegów (PL); Rusak, Agnieszka, 50-512 Wroclaw (PL); Krawczynska, Klaudia, 50-304 Wroclaw (PL); Górnicki, Tomasz, 57-100 Gesiniec (PL)
(74) Representative: Witek, Rafal

(57) **Abstract**

Novel derivatives of 4,6-dimethylisothiazolo[5,4-b]pyridine conjugated with an N-acetylhydrazone moiety according to formula (1), suitable for use in the treatment or prevention of cancer, especially malignant melanoma, and a method for their preparation have been disclosed.

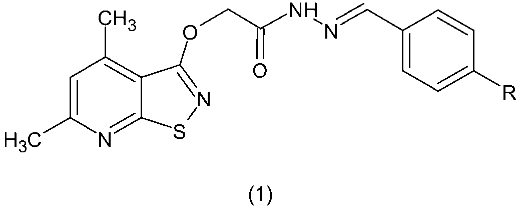

## Description

Compounds that contain the N-acylhydrazone or hydrazide-hydrazone moiety are an important group of derivatives among which novel drug candidates are sought. Particularly numerous scientific reports concern the anticancer activity of compounds containing the *N*-acylhydrazone moiety, the activity of which is based on a selective effect on various stages of carcinogenesis, including disruption of signaling pathways necessary for tumor growth or induction of apoptosis. Literature reports show that *N*-acylhydrazone derivatives have been studied as potential inhibitors of tyrosine kinases of the vascular endothelial growth factor (VEGF) [Sahar M. Abou-Seri, Amal A.M. Eissa, Mohamed G.M. Behery, Farghaly A. Omar, Bioorg. Chem. 2021, 116, 105334] and the epidermal growth factor (EGFR) [Ahmed NM, Youns MM, Soltan MK, Said AM, Molecules 2021; 26(7):1838], carbonic anhydrase inhibitors [Mohammed M.S. Wassel, Ahmed Ragab, Gameel A.M. Elhag Ali, Ahmed B.M. Mehany, Yousry A. Ammar, J. Mol. Struct. 2021, 1223, 128966], compounds that interfere with the tubulin polymerization process [Brullo C., Rapetti F., Alfei S., Maric I., Rizzelli F., Mapelli M., Rosano C., Viale M., Bruno O., ChemMedChem 2020, 15, 961] as well as proapoptotic compounds activating procaspase-3 to caspase-3 [Putt KS, Chen GW, Pearson JM, Sandhorst JS, Hoagland MS, Kwon JT, Hwang SK, Jin H, Churchwell MI, Cho MH, Doerge DR, Helferich WG, Hergenrother PJ., Nat Chem Biol. 2006 Oct;2(10):543-50].

On the other hand, as compounds that could be potentially used in cancer therapy, derivatives containing aza-heterocyclic rings in their structure, including a pyridine ring [Mohamed E.A., Ismail N.S.M., Hagras M., Hanan R. Futur. J Pharm. Sci. 2021 7, 24], as well as compounds based on a condensed benzisothiazole system [Coviello V., Marchi B., Sartini S., Quattrini L., Marini a.m., Simorini F., Taliani S., Salerno S., Orlandi P., Fioravanti A., Desidero T., Vullo D., Settimo F., Supuran C.T., Bocci G., La Motta C., J. Med. Chem. 2016, 59 (13), 6547-6552] are often studied.

The objective of the invention is to provide novel compounds having anticancer activity, suitable for use in the treatment or prevention of cancer, especially malignant melanoma.

The object of the invention is novel derivatives of 4,6-dimethylisothiazolo[5,4-*b*]pyridine conjugated with the *N*-acetylhydrazone moiety, represented by general formula 1: 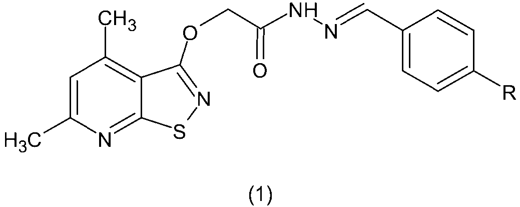 wherein R is a substituent selected from groups -SCH₃, -OCH₃, -N(CH₃)₂ or OH.

Preferably, it is selected from compound 4i of formula 2 wherein R is the -SCH₃ group, compound 4j of formula 3 wherein R is the -OCH₃ group, compound 4I of formula 4 wherein R is the -N(CH₃)₂ group or compound 4n of formula 5 wherein R is the -OH group.

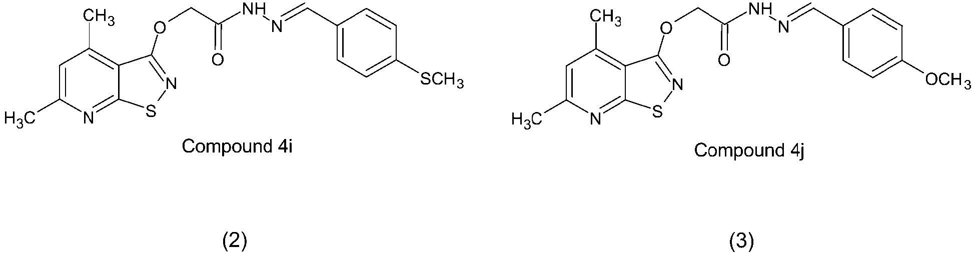

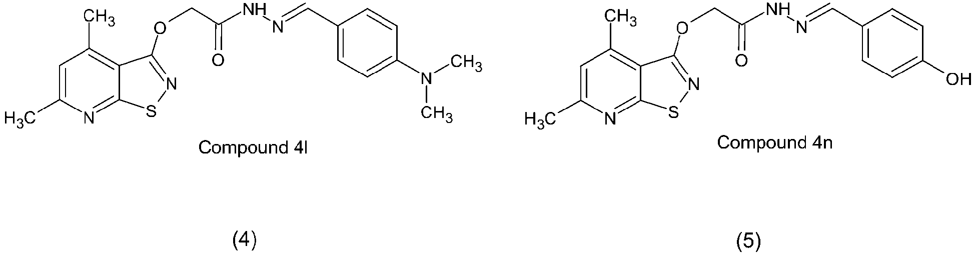

In the context of the invention, deaza analogs of isothiazolopyridine, which is the parent skeleton of the compounds presented by general formula (1), may be considered as benzisothiazole derivatives.

A further object of the invention is a method for the preparation of novel *N*-acetylhydrazone derivatives of 4,6-dimethylisothiazolo[5,4-b]pyridine of general formula 1, wherein R is groups -SCH₃, - OCH₃, -N(CH₃)₂ or -OH, comprising the steps of:
a) a reaction of 4,6-dimethylisothiazolo[5,4-*b*]pyridin-3(2*H*)-one with methyl chloroacetate in refluxing acetone in the presence of potassium carbonate;
b) a reaction of 2-[(4,6-dimethylisothiazolo[5,4-*b*]pyridin-3-yl)oxo]acetic acid methyl ester with hydrazine hydrate under reflux in ethanol, followed by cooling and filtration to afford 2-[(4,6-dimethylisothiazolo[5,4-*b*]pyridin-3-yl)oxo]acetic acid hydrazide;
c) a reaction of the previously obtained 2-[(4,6-dimethylisothiazolo[5,4- *b*]pyridin-3-yl)oxo]acetic acid hydrazide with 4-methylsulfanylbenzaldehyde for compound **4i**, 4-methoxybenzaldehyde for compound **4j,** 4-dimethylaminobenzaldehyde for compound **4l,** or 4-hydroxybenzaldehyde for compound **4n,** in refluxing ethanol in the presence of a catalytic amount of acetic acid;
d) crystallization of the precipitate from an ethanol/water mixture (4:1).

A further object of the invention are novel derivatives of 4,6-dimethylisothiazolo[5,4-b]pyridine of general formula 1, as defined above, especially derivatives in which R represents groups: -SCH₃ (compound **4i** of formula 2), -OCH₃ (compound **4j** of formula 3), -N(CH₃)₂ (compound **4l** of formula 4), or -OH (compound **4n** of formula 5) for use in the treatment of cancer, especially in the treatment of malignant melanoma.

Biological studies to confirm the anticancer activity of the new derivatives of 4,6-dimethylisothiazolo[5,4-b]pyridine of general formula 1 were performed using human-derived cell lines, both normal (skin fibroblasts, NHDF) and neoplastic (melanoma cell line, A375). Anticancer activity was assessed in the MTT assay, a quantitative colorimetric method used to determine mitochondrial energy transformation activity. Under the test conditions, the yellow water-soluble 3-(4,5-dimethylthiazol-2-yl)-2,5-diphenyltetrazolium bromide (MTT) salt is reduced to purple, water-insoluble formazan crystals by mitochondrial dehydrogenase. The quantity of generated crystals is proportional to the oxidative activity of the mitochondria, and under strictly defined test conditions, to the number of viable cells. In addition to the tested compounds, to be able to compare their biological activity to the anticancer drugs currently used in therapeutic regimens, the effect on the mitochondrial activity of 5-fluorouracil (5-FU), a commercially available active substance, was also assessed. Based on the measurement of absorbance in the MTT assay, the viability of cell cultures was calculated, and then the IC₅₀ value was determined, i.e., the concentration of a specific inhibitory substance at which the survival of the tested cells was inhibited by 50%.

The IC₅₀ results for the said compounds and the reference drug are presented in Tables 1-3 (after 24-, 48-, and 72-hour cell exposure to the tested compounds). Under the test conditions, compounds **4i, 4j, 4l,** and **4n** did not show cytotoxic activity against normal skin fibroblast cells (NHDF) in the range of cytotoxic activity concentrations for melanoma cancer cells (A375). Compounds **4i** after 24- and 48-hour incubation and **4n** after 72-hour incubation showed the highest selectivity against melanoma. It is noteworthy that compound **4i** was characterized by higher cytotoxic activity against melanoma neoplastic cells at all time intervals compared to the reference drug.

**Table 1. Cytotoxicity of compounds 4i, 4j, 4l, 4n, and 5-FU measured by MTT assay after 24 hours of incubation.**

| **Compound name** | **IC₅₀ (µM)** | |
|---|---|---|
| | **A375** | **NHDF** |
| **4i** | 1.785 | 96.006 |
| **4j** | 0.859 | 23.370 |
| **4l** | 5.628 | 91.676 |
| **4n** | 3.018 | 59.148 |
| **5-FU** | 16.648 | 32.098 |

**Table 2. Cytotoxicity of compounds 4i, 4j, 4l, 4n, and 5-FU measured by MTT assay after 48 hours of incubation.**

| **Compound name** | **IC₅₀ (µM)** | |
|---|---|---|
| | **A375** | **NHDF** |
| **4i** | 1.033 | 29.559 |
| **4j** | 8.825 | 23.548 |
| **4l** | 4.445 | 36.068 |
| **4n** | 2.285 | 56.736 |
| **5-FU** | 4.067 | 85.958 |

**Table 3. Cytotoxicity of compounds 4i, 4j, 4l, 4n, and 5-FU measured by MTT assay after 72 hours of incubation.**

| **Compound name** | **IC₅₀ (µM)** | |
|---|---|---|
| | **A375** | **NHDF** |
| **4i** | 1.314 | 47.179 |
| **4j** | 3.166 | 28.062 |
| **4l** | 5.923 | 22.873 |
| **4n** | 2.271 | 93.608 |
| **5-FU** | 1.813 | 104.510 |

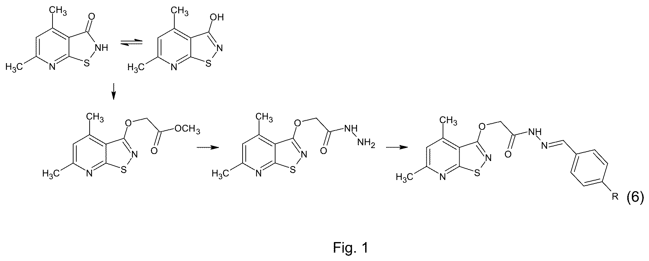

The object of the invention is shown in more detail in the embodiments, formulas (1-5), and based on the reaction diagram shown in Fig. 1.

### General procedure for the synthesis of 2-[(4,6-dimethylisothiazolo[5,4-b]pyridin-3-yl)oxo]acetic acid methyl ester (step 1)

To a suspension of K₂CO₃ (2 eq.) in acetone (30 mL) 4,6-dimethylisothiazolo[5,4-*b*]pyridin-3(2H)-one (1 eq.) was added, and subsequently methyl chloroacetate (2 eq.) was gradually added dropwise. The resulting suspension was stirred with a magnetic stirrer at the boiling point of acetone (329 K) for 4 hours. The mixture was cooled, poured onto ice, and the resulting precipitated **2-[(4,6-dimethylisothiazolo[5,4-*b*]pyridin-3-yl)oxo]acetic acid methyl ester** was filtered and washed with water. The crude product was used in the next reaction step.

### General procedure for the synthesis of 2-[(4,6-dimethylisothiazolo[5,4-b]pyridin-3-yl)oxo]acetic acid hydrazide (step 2)

To a suspension of 2-[(4,6-dimethylisothiazolo[5,4-*b*]pyridin-3-yl)oxo]acetic acid methyl ester (1 eq.) in ethanol (35 mL), hydrazine hydrate (10 eq.) was added. The resulting solution was stirred with a magnetic stirrer at the boiling point of the solvent (351,52 K) for 3 hours. The mixture was cooled and left overnight. The resulting precipitated **2-[(4,6-dimethylisothiazolo[5,4-*b*]pyridin-3-yl)oxo]acetic acid hydrazide** was filtered and washed with ethanol. The crude product was used in the next reaction step.

### Procedure for the synthesis of compounds 4i, 4j, 4l and 4n (step 3)

To a suspension **of 2-[(4,6-dimethylisothiazolo[5,4-*b*]pyridin-3-yl)oxo]acetic acid hydrazide** (1 eq.) in 40 mL of ethanol, 2.5 mL of glacial acetic acid was added and stirred with a magnetic stirrer for 5 minutes. Subsequently, 4-methylsulfanylbenzaldehyde for compound **4i,** 4-methoxybenzaldehyde for compound **4j,** 4-dimethylaminobenzaldehyde for compound **4l,** or 4-hydroxybenzaldehyde for compound **4n** were added to the suspension. The stirring was continued for 4 h at the boiling point of the solvent (351,52 K). The resulting product was filtered, washed with ethanol and crystallized from ethanol/water to yield ***E*-2-[(4,6-dimethylisothiazolo[5,4-*b*]pyridin-3-yl)oxo]-N'-[(4-methylsulfanylphenyl)methylidene]acetohydrazide** (compound **4i**) with a melting point of 484-487 K with a yield of 91% of theoretical efficiency, ***E*-2-[(4,6-dimethylisothiazolo[5,4-*b*]pyridin-3-yl)oxo]-N'-[(4-methoxyphenylmethylidene]acetohydrazide** (compound **4j**) with a melting point of 477-478 K with a yield of 94% of theoretical efficiency, ***E*-2-[(4,6-dimethylisothiazolo[5,4-*b*]pyridin-3-yl)oxo]-N'-[(4-dimethylaminophenyl)methylidene]acetohydrazide** (compound **4l**) with a melting point of 503-507 K with a yield of 73% theoretical efficiency and ***E*-2-[(4,6-dimethylisothiazolo[5,4-b]pyridin-3-yl)oxo]-N'-[(4-hydroxy-phenyl)methylidene]acetohydrazide** (compound **4l**) with a melting point of 555-557 K with a yield of 95% theoretical efficiency.

The properties of the new compounds of analytical grade are characterized in the table below:

**Table 4. Molecular formulas and spectral properties of compounds 4i, 4j, 4l, and 4n.**

| Compound number | Molecular formula | FT-IR (ATR) (v, cm⁻¹) | ¹H NMR (δ, ppm) (DMSO-d₆) | ¹³C NMR (δ, ppm) (DMSO-d₆) |
|---|---|---|---|---|
| 4i | C₁₈H₁₈N₄O₂S₂ | 3196 (NH), 3069 (C-H arom.), 2923 (C-H aliph.), 1676 (C=O) | 2.58 (s, 3H, CH_{3-pyridine}), 2.69 (s, 3H, CH₃-_{pyridine}), 5.05, 5.52 (2 s, 2H, CH₂), 7.22 (s, 1H, H_{-pyridine}), 7.27-7.30 (d, 2H, ArH, J=8.4Hz), 7.60-7.63 (d, 2H, ArH, J=8.4Hz) 7.95, 8.17 (s, 1H, NH), 11.61 (s, 1H, N=CH) | 14.77, 18.91, 24.53, 65.48, 66.23, 115.95, 122.26, 126.12, 127.73, 128.01, 130.81, 141.35, 144.15, 146.08, 147.60, 161.53, 163.90, 168.50, 171.76 |
| 4j | C₁₈H₁₈N₄O₃S | 3192 (NH), 3061 (C-H arom.), 2920 (C-H aliph.), 1680 (C=O) | 2.58 (s, 3H, CH_{3-pyridine}), 2.69 (s, 3H, CH₃-_{pyridine}), 3.78 (s, 3H, OCH₃), 5.04, 5.52 (2 s, 2H, CH₂), 6.97-7.00 (d, 2H, ArH, J=9.0Hz), 7.22 (s, 1H, H_{-pyridine}), 7.6-7.64 (d, 2H, ArH, J=9.0Hz), 7.94, 8.18 (s, 1H, NH), 11.52 (s, 1H, N=CH) | 18.86, 24.48, 55.72, 65.45, 66.26, 114.73, 115.91, 122.20, 126.94, 128.88, 129.20, 144.32, 146.00, 147.88, 161.19, 161.44, 161.64, 163.71, 168.31, 171.80 |
| 4l | C₁₉H₂₁N₅O₂S | 3189 (NH), 3076 (C-H arom.), 2920 (C-H aliph.), 1687 (C=O) | 2.58 (s, 3H, CH_{3-pyridine}), 2.69 (s, 3H, CH₃-_{pyridine}), 2.94 (s, 6H, N(CH₃)₂), 3.78 (s, 3H, OCH₃), 5.02, 5.49 (2 s, 2H, CH₂), 6.70-6.73 (d, 2H, ArH, J=9.0Hz), 7.21 (s, 1H, H_{-pyridine}), 7.46-7.49 (d, 2H, ArH, J=9.0Hz), 7.86, 8.07 (s, 1H, NH), 11.34, 11.35 (s, 1H, N=CH) | 18.91, 24.53, 65.55, 66.32, 112.25, 116.01, 121.73, 122.29, 128.61, 128.92, 145.27, 146.11, 148.85, 151.90, 161.50, 161.75, 163.37, 167.97, 171.83 |
| 4n | C₁₇H₁₆N₄O₃S | 3190 (NH), 3070 (C-H arom.), 2922 (C-H aliph.), 1682 (C=O) | 2.60 (s, 3H, CH_{3-pyridine}), 2.71 (s, 3H, CH₃-_{pyridine}), 5.06, 5.52 (2 s, 2H, CH₂), 6.82-6.84 (d, 2H, ArH, J=8.4Hz), 7.23 (s, 1H, H-_{pyridine}), 7.52-7.54 (d, 2H, ArH, J=8.4Hz), 7.92, 8.13 (s, 1H, NH), 9.93 (s, 1H, OH), 11.47 (s, 1H, N=CH) | 18.92, 24.53, 65.54, 66.26, 115.95, 116.13, 122.31, 125.38, 129.14, 129.35, 144.77, 146.08, 148.28, 159.77, 161.56, 161.75, 163.63, 168.23, 171.80 |

## Claims

1. A compound of the general formula 1: 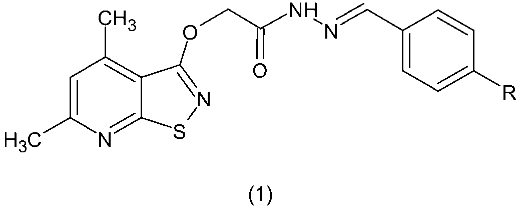 wherein R is a substituent selected from groups -SCH₃, -OCH₃, -N(CH₃)₂ or OH.

2. The compound of Claim 1 **characterized in that** it is selected from the group comprising:
- compound 4i of formula 2, wherein R is the -SCH₃ group,
- compound 4j of formula 3, wherein R is the -OCH₃ group,
- compound 4l of formula 4, wherein R is the -N(CH₃)₂ group or
- compound 4n of formula 5, wherein R is the -OH group.

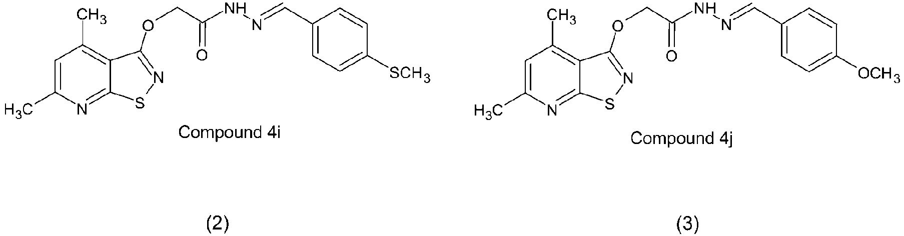

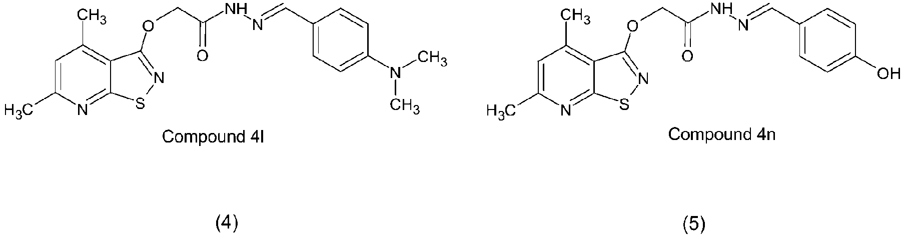

3. A method for the preparation of novel N-acetylhydrazone derivatives of 4,6-dimethylisothiazolo[5,4-b]pyridine of general formula 1, wherein R is groups -SCH₃, -OCH₃, -N(CH₃)₂ or -OH, **characterized in that**:
a) a reaction of 4,6-dimethylisothiazolo[5,4-*b*]pyridin-3(2*H*)-one with methyl chloroacetate is conducted in refluxing acetone in the presence of potassium carbonate;
b) subsequently, a reaction of 2-[(4,6-dimethylisothiazole[5,4-*b*]pyridin-3-yl)oxo]acetic acid methyl ester with hydrazine hydrate is performed under reflux in ethanol, with cooling and filtration of the formed precipitate of 2-[(4,6-dimethylisothiazole[5,4-*b*]pyridin-3-yl)oxo]acetic acid hydrazide;
c) subsequently, a reaction of the 2-[(4,6-dimethylisothiazole[5,4-*b*]pyridin-3-yl)oxo]acetic acid hydrazide obtained in the previous step is performed with a benzaldehyde derivative selected from: 4-methylsulfanylbenzaldehyde, 4-methoxybenzaldehyde, 4-dimethylaminobenzaldehyde, or 4-hydroxybenzaldehyde,
d) the formed precipitate is crystallized from an ethanol/water mixture (4:1).

4. The compound of Claims 1-2 for use in the treatment or prevention of cancer, especially
malignant melanoma.
